# EUROPÄISCHE PATENTANMELDUNG

(11) **EP 1 074 227 A2**
(43) Veröffentlichungstag der Anmeldung: **07.02.2001**
(21) Anmeldenummer: 00250261.5
(22) Anmeldetag: 03.08.2000
(51) Int. Cl.: A61F 2/06

(54) **Stent für Gefässverzweigungen**

(30) Priorität: 06.08.1999 DE 19938377
(71) Anmelder: BIOTRONIK Mess- und Therapiegeräte GmbH & Co Ingenieurbüro Berlin, 12359 Berlin (DE)
(72) Erfinder: Loos, Hartmut, 3090 Overijse (BE); Kranz, Curt, 14163 Berlin (DE)
(74) Vertreter: Eisenführ, Speiser & Partner

(57) **Zusammenfassung**

Stent, insbesondere Koronarstent, zur Implantation im Bereich von Gefäßverzweigungen (4), mit einem rohrförmigen Mantel (2) mit einem proximalen und einem distalen Ende, wobei im implantierten Zustand das proximale Ende proximal und das distale Ende distal der Gefäßverzweigung (4) angeordnet ist, und wenigstens einem am Umfang des Mantels (2) angeordneten, zur Freigabe eines Durchgangs in den abzweigenden Gefäßast (3.1) vorgesehenen Verzweigungsabschnitt (5), der im implantierten Zustand im Bereich der Gefäßverzweigung angeordnet ist, wobei der Verzweigungsabschnitt (5) wenigstens ein zum Abstützen des Gefäßübergangs vorgesehenes, im wesentlichen radial aus der Mantelfläche herausklappbares erstes Stützelement (6) umfaßt, das sich aus einer ersten Richtung in den Verzweigungsabschnitt (5) hinein erstreckt, und wobei der Verzweigungsabschnitt (5) wenigstens ein zum Abstützen des Gefäßübergangs vorgesehenes, im wesentlichen radial aus der Mantelfläche herausklappbares zweites Stützelement (7) umfaßt, das sich aus einer der ersten Richtung im wesentlichen entgegengesetzten zweiten Richtung in den Verzweigungsabschnitt (5) hinein erstreckt.

## Beschreibung

Die Erfindung betrifft einen Stent, insbesondere Koronarstent, zur Implantation im Bereich von Gefäßverzweigungen, mit einem rohrförmigen Mantel mit einem proximalen und einem distalen Ende.

Bei einem Stent handelt es sich um ein intraluminales Stützelement. Ein solches Stützelement dient zum Abstützen der Wandung eines in der Regel verengten Gefäßes des menschlichen oder tierischen Körpers, um dieses in einer aufgeweiteten Stellung zuhalten. Derartige Stents finden als sog. Koronarstents häufig Anwendung im Bereich verengter Herzkranzgefäße.

Bekannte Stents bestehen dabei in der Regel aus einem rohrförmigen Mantel mit einem proximalen und einem distalen Ende, der in einem nicht expandierten Zustand an die aufzuweitende Stelle im Gefäß herangeführt wird. Befindet er sich in der gewünschten Position, wird der Mantel in geeigneter Weise radial auf einen Durchmesser expandiert, bei dem er die umliegende Gefäßwand auf den gewünschten Durchmesser aufweitet. Der Stent verbleibt hiernach von selbst in diesem Zustand, um das Gefäß dauerhaft aufgeweitet zu halten.

Häufig ist es erforderlich, den Stent im Bereich von Gefäßverzweigungen zu implantieren. Hierzu sind Stents bekannt, bei denen im implantierten Zustand das proximale Ende proximal der Gefäßverzweigung und das distale Ende distal der Gefäßverzweigung angeordnet ist. Sie weisen weiterhin wenigstens einem am Umfang des Mantels angeordneten Verzweigungsabschnitt auf, der im implantierten Zustand im Bereich der Gefäßverzweigung angeordnet ist. Dieser Verzweigungsabschnitt wird beim Implantieren in der Regel so verformt, daß er einen Durchgang in den abzweigenden Gefäßast freigibt.

Ein solcher Stent ist beispielsweise aus der europäischen Patentanmeldung EP 0 904 745 A2 bekannt. Bei diesem wird oder werden zur Freigabe des Durchgangs in den abzweigenden Gefäßast, ein oder mehrere in Umfangsrichtung des Stents aneinandergrenzende, sich aus einer ersten Richtung, hier aus Richtung des einen Endes des Mantels, in den Verzweigungsabschnitt hinein erstreckende erste Stützelemente durch plastische Verformung im wesentlichen radial aus der Mantelfläche heraus in den abzweigenden Gefäßast geklappt.

Hierbei besteht jedoch das Problem, daß der Gefäßübergang der Verzweigung nur unzureichend abgestützt wird. Bei dem Gefäßübergang handelt es sich zum einen um einen sehr empfindlichen Bereich des Gefäßes, der besonders beansprucht ist, wenn lediglich in einem Gefäßast ein Stent implantiert ist. Zum anderen ist die Aufrechterhaltung seiner natürlichen Geometrie aus strömungstechnischen Gesichtspunkten von Bedeutung, da nur so die normale Zirkulation wiederhergestellt werden kann und z. B. die Bildung von Ablagerungen durch ungünstige Strömungsverhältnisse vermieden werden kann.

Zwar ermöglicht das erste Stützelement des bekannten Stents bereits eine gewisse Abstützung des Gefäßüberganges. Diese ist jedoch nur einseitig und führt daher unter Umständen zu einer unerwünschten unnatürlichen Verformung und Belastung des Gefäßüberganges.

Der Erfindung liegt deshalb die Aufgabe zugrunde, einen Stent der eingangs genannten Art zur verbesserten Abstützung des Gefäßüberganges im Bereich einer Gefäßverzweigung zur Verfügung zu stellen.

Die Aufgabe wird, ausgehend von einem Stent der eingangs genannten Art dadurch gelöst, daß der Verzweigungsabschnitt wenigsten ein zum Abstützen des Gefäßübergangs vorgesehenes, im wesentlichen radial aus der Mantelfläche herausklappbares zweites Stützelement umfaßt, das aus einer zweiten, anderen als der ersten Richtung in den Verzweigungsabschnitt hinein erstreckt.

Die Erfindung schließt die technische Lehre ein, daß eine verbesserte Abstützung des Gefäßüberganges zu erzielen ist, wenn der Verzweigungsabschnitt wenigstens ein zweites zum Abstützen des Gefäßübergangs vorgesehenes, im wesentlichen radial aus der Mantelflache herausklappbares Stützelement umfaßt, das sich aus einer zur ersten Richtung im wesentlichen entgegengesetzten Richtung in den Verzweigungsabschnitt hinein erstreckt.

Die beiden Stützelemente können hierbei nach Art einer Saloon-Tür in den abzweigenden Gefäßast geklappt werden und stützen somit den Gefäßübergang von zwei gegenüberliegenden Punkten ab. Hierdurch ist eine gleichmäßigere Einleitung der Lasten in den empfindlichen Gefäßübergang sichergestellt, wie sie beispielsweise gerade bei den im Bereich der Koronargefäße starken Gefäßbewegungen quer zur Ebene der Verzweigung auftreten.

Bei der ersten Richtung kann es sich um eine beliebige Richtung handeln. So ist es beispielsweise möglich, daß die erste Richtung vom distalen Ende des Stents her parallel zu dessen Längsachse verläuft. Es ist jedoch auch möglich, daß die erste Richtung in einer Umfangsrichtung des Stents verläuft.

Vorzugsweise ist wenigstens ein drittes, zu dem ersten oder zweiten Stützelement quer zur ersten Richtung benachbart angeordnetes Stützelement vorgesehen. Die Stützabschnitte können gleichmäßig verteilt sein und sich beispielsweise aus um jeweils 120° zueinander versetzten Richtungen in den Verzweigungsabschnitt hinein erstrecken. Hierdurch wird eine noch gleichmäßigere Abstützung sichergestellt. Diese kann noch zusätzlich verbessert werden, wenn weiter vorzugsweise jeweils zu dem ersten und zweiten Stützelement ein quer zur ersten Richtung benachbart angeordnetes viertes Stützelement vorgesehen ist. Es versteht sichim übrigen, daß jeweils auch noch weitere quer zur ersten Richtung benachbart angeordnete Stützelemente vorgesehen sein können.

Das erste und zweite Stützelement können dabei so angeordnet sein, daß sie im Ausgangszustand quer zur ersten Richtung aneinander angrenzen, d. h. über eine bestimmte Länge aneinander entlang verlaufen. Vorzugsweise ist das zweite Stützelement mit dem ersten Stützelement in der ersten Richtung im wesentlichen fluchtend angeordnet. Hierdurch ist eine einfache Positionierung des Stents im Bereich der Gefäßverzweigung mit einem abzweigenden Gefäßast kleineren Durchmessers möglich. Es ist hierbei lediglich erforderlich, die beiden freien Enden der Stützelemente etwa in der Mitte des Durchganges der Verzweigung zu plazieren, was beispielsweise unter Zuhilfenahme eines zwischen den freien Enden hindurchgeführten Führungsdrahtes in einfacher Weise möglich ist.

Bei bevorzugten Ausführungen des erfindungsgemäßen Stents sind die Stützelemente langgestreckt ausgebildet, wodurch zum einen ein einfaches Verformen in ihre Stützstellung möglich ist. Zum anderen ermöglichen derartige Stützelemente einen Einsatz bei Gefäßverzweigungen mit unterschiedlichsten Durchmessern des abzweigenden Gefäßastes. So kann bei kleineren Durchmessern des abzweigenden Gefäßastes nur der freie Endbereich des jeweiligen Stützelements zur Abstützung des Gefäßüberganges herangezogen werden, während bei größeren Durchmessern gegebenenfalls das Stützelement über seine gesamte Länge in den abzweigenden Gefäßast hineinreicht. Hierdurch wird dann zudem eine hohe Stützlänge und damit eine gute Abstützung erzielt.

Bei weiteren bevorzugten Varianten der Erfindung sind die Stützelemente von sich im wesentlichen parallel zur ersten Richtung erstreckenden Stegelementen gebildet, die sich durch eine besonders einfache Handhabung beim Verformen in ihre Stützstellung auszeichnen.

Vorzugsweise ist der Verlauf der Stegelemente dabei im wesentlichen nach Art einer Haarnadel ausgebildet, wobei der Wendebereich das freie Ende des Stützelements bildet. Hierdurch wird zum einen ein stabiles Stützelement erzielt. Zum anderen ist bei dieser Gestaltung eine besonders günstige Konfiguration möglich, bei der die Schenkel des Stützelements derart am Mantel angeschlossen sind, daß sie sich bei der Expansion des Stents voneinander entfernen. Dies führt zum einen zu einer stabilen Stützgeometrie, zum anderen zu weiter von einander entfernten und damit gleichmäßiger verteilten Abstützpunkten der Gefäßwandung. Weitere bevorzugte Ausführungen des erfindungsgemäßen Stents zeichnen sich dadurch aus, daß quer zur ersten Richtung aneinander angrenzende Stützelemente an ihrem freien Ende über wenigstens ein Verbindungselement verbunden sind, wodurch eine großflächigere Abstützung des Gefäßüberganges erzielt wird. Vorzugsweise ist das Verbindungselement bogenartig ausgebildet, so daß beim Verformen der Stützelemente in ihre Stützstellung ein Längenausgleich zwischen den freien Enden erfolgen kann.

Bei weiteren vorteilhaften Varianten der Erfindung sind die Stützelemente derart ausgebildet und angeordnet, daß der Verzweigungsabschnitt im implantierten Zustand bei zum Abstützen des Gefäßübergangs radial aus der Mantelfläche geklappten Stützelementen einen der Kontur des Gefäßübergangs im wesentlichen angepaßten Durchgang in der Mantelfläche freigibt. Hierdurch ist eine besonders gleichmäßige Abstützung des Übergangsbereiches sichergestellt.

Besonders günstige, weil einfach zu positionierende Weiterbildungen des erfindungsgemäßen Stents, weisen eine Anzahl Verzweigungsabschnitte auf. Zum Positionieren genügt es dann in der Regel einen beliebigen der Verzweigungsabschnitte im Bereich der Gefäßverzweigung zu plazieren.

Es ist aber auch möglich, Verzweigungsabschnitte für Durchgänge unterschiedlicher Abmessungen vorzusehen, die sich für den Einsatz im Bereich von abzweigenden Gefäßästen unterschiedlichen Durchmessers eignen.

Vorzugsweise ist wenigstens ein Teil der Verzweigungsabschnitte in einem Ringbereich des Mantels in Umfangsrichtung des Mantels verteilt angeordnet, so daß zum Positionieren eines Verzweigungsabschnitts in Bereich der Verzweigung die Winkellage des Stents um seine Längsachse nur um einen vergleichsweise geringen Maximalbetrag verändert werden muß. Dieser Maximalbetrag ist dabei besonders gering, wenn die Verzweigungsabschnitte in dem Ringbereich unmittelbar aneinander anschließend angeordnet sind.

Die vorliegende Erfindung betrifft weiterhin ein Verfahren zum Implantieren eines erfindungsgemäßen Stents, welches sich dadurch auszeichnet, daß die Stützelemente nach dem Expandieren des Mantels nach Art einer Saloon-Tür durch ein an den Verzweigungsabschnitt herangeführtes Betätigungsmittel radial aus der Mantelfläche heraus in die Gefäßverzweigung hinein geklappt werden. Als Betätigungsmittel wird hierbei vorzugsweise ein Führungsdraht verwendet. Vorzugsweise wird dabei als Betätigungsmittel ein Führungsdraht verwendet, der auch zum Positionieren des Stents vor der Expansion dient, so daß sich das Heranführen oder Vorsehen eines gesonderten Betätigungsmittels erübrigt. Weiter vorzugsweise wird zum Expandieren des Stents ein Ballonkatheter verwendet, der an seinem distalen Ende einen Ballon mit einer proximalen und einer davon beabstandeten distalen Kammer aufweist, zwischen denen der das Betätigungsmittel bildende Führungsdraht austritt. Hierdurch ist eine besonders einfache und schnelle Implantation des Stents möglich.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1: eine schematische Ansicht einer bevorzugten Ausführung des erfindungsgemäßen Stents im implantierten Zustand;
- Figur 2: eine Abwicklung des Mantels einer weiteren Ausführung des erfindungsgemäßen Stents;
- Figur 3: eine Abwicklung des Mantels einer anderen Ausführung des erfindungsgemäßen Stents.

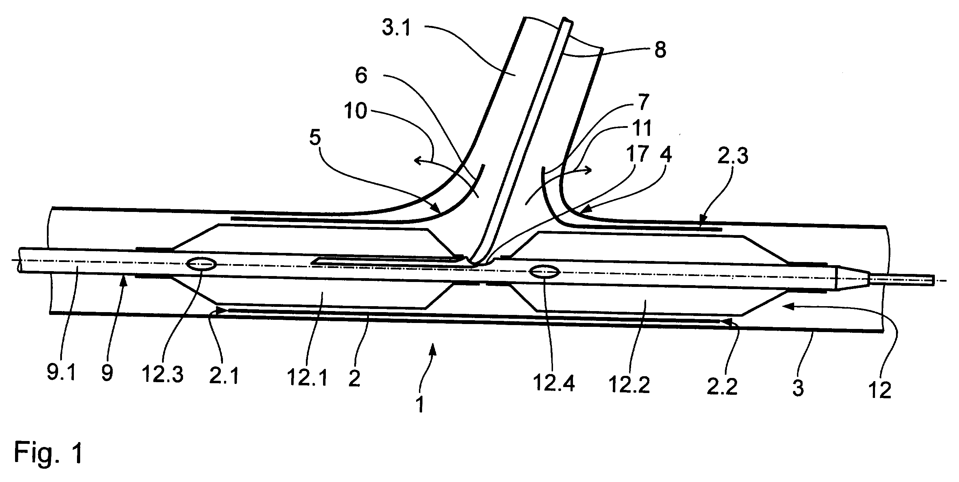

Figur 1 zeigt eine schematische Ansicht eines erfindungsgemäßen Stents 1 mit einem rohrförmigen Mantel 2, der ein proximales Ende 2.1 und ein distales Ende 2.2 aufweist. Der Stent 1 ist in einem in ein Blutgefäß 3 implantierten Zustand gezeigt. Er befindet sich im Bereich einer Gefäßverzweigung 4, wobei sein proximales Ende 2.1 proximal der Gefäßverzweigung 4 und sein distales Ende 2.2 distal der Gefäßverzweigung 4 angeordnet ist.

Der Stent 1 weist einen Verzweigungsabschnitt 5 auf, der im gezeigten implantierten Zustand im Bereich der Gefäßverzweigung 4 angeordnet ist. Der Verzweigungsabschnitt 5 umfaßt zum Abstützen des Gefäßüberganges 4 ein langgestrecktes erstes Stützelement 6 und ein langgestrecktes zweites Stützelement 7. Im hier nicht dargestellten Ausgangszustand des Stents 1 liegen das erste Stützelement 6 und das zweite Stützelement 7 in der Mantelfläche 2.3 des Mantels 2. Das erste Stützelement 6 erstreckt sich dabei in einer ersten Richtung in den Verzweigungsabschnitt 5 hinein. Diese erste Richtung verläuft aus Richtung des proximalen Endes 2.1 des Stents 1 parallel zu dessen Längsachse. Das zweite Stützelement 7 erstreckt sich in einer zur ersten Richtung entgegengesetzten zweiten Richtung in den Verzweigungsabschnitt 5 hinein, d. h. aus Richtung des distalen Endes 2.2 parallel zur Längsachse des Stents 1.

Die Stützelemente 6 und 7 werden mittels eines ersten Führungsdrahtes 8 des Ballonkatheters 9 nach Art einer Saloon-Tür in Richtung der Pfeile 10 bzw. 11 radial aus der Mantelfläche 2.3 heraus in den abzweigenden Gefäßast 3.1 hinein gebogen. Im hier nicht dargestellten, fertig implantierten Zustand liegen das erste und zweite Stützelement 6 und 7 zur Abstützung des Gefäßüberganges 4 vollständig an dessen Wandung an.

Der Mantel 2 des Stents 1 kann dabei in beliebiger bekannter Weise ausgebildet sein. So kann er wie im gezeigten Beispiel mit einer plastisch deformierbaren Struktur, beispielsweise einer bekannten gitterartigen Stegstruktur, ausgebildet sein, die mittels des Ballons 12 des Ballonkatheters 9 radial in den gezeigten auf geweiteten Zustand verformt wird und nach Entfernen des Ballons 12 in diesem Zustand verbleibt, um das Gefäß 3 dauerhaft aufgeweitet zu halten.

Es versteht sich jedoch, daß der Mantel auch als sog. selbstexpandierender Typ ausgebildet sein kann, der sich durch Rückstellen einer vorherigen elastischen radialen Kompression von selbst in einen Zustand verformt, in dem er das Gefäß dauerhaft aufgeweitet hält.

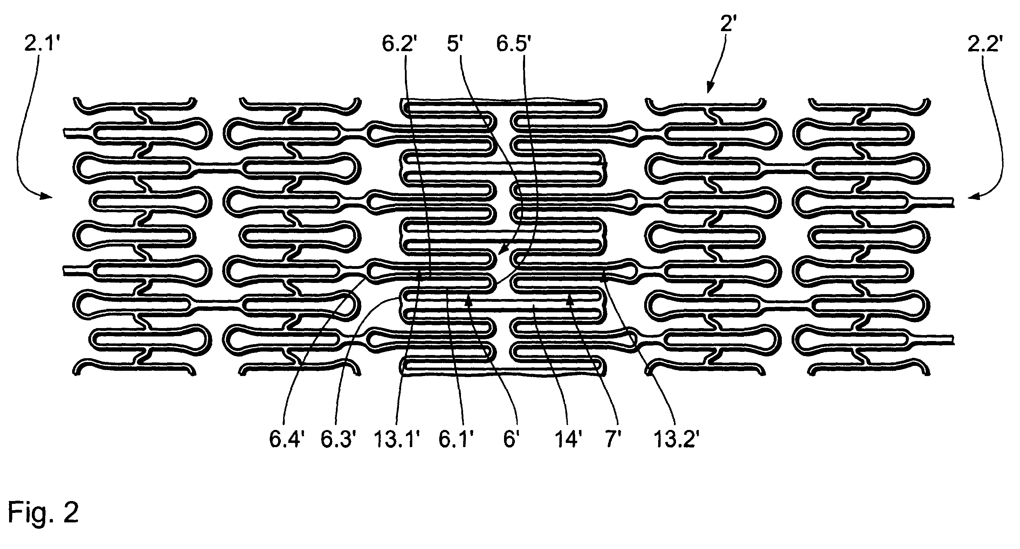

Figur 2 zeigt eine Abwicklung des Mantels 2' eines erfindungsgemäßen Stents 1', der aus einer Struktur stegartiger Elemente besteht. Der Mantel 2' weist dabei eine Anzahl in seiner Umfangsrichtung aneinander angrenzender Verzweigungsabschnitte 5' auf.

Der jeweilige Verzweigungsabschnitt 5' umfaßt ein erstes Stützelement 6', ein in der ersten Richtung, d. h. in Längsrichtung des Mantels 2', fluchtend dazu angeordnetes zweites Stützelement 7' sowie jeweils ein zu dem ersten bzw. zweiten Stützelement 6' bzw. 7' quer zur ersten Richtung, d. h. in Umfangsrichtung des Mantels 2', benachbart angeordnetes drittes Stützelement 13.1' bzw. 13.2'. Weiterhin wird der Verzweigungsabschnitt 5' in Umfangsrichtung des Mantels 2' zu beiden Seiten von einem in Richtung der Längsachse des Mantels 2' verlaufenden Steg 14' begrenzt.

Die Stützelemente 6', 7', 13.1' und 13.2' sind jeweils von sich im wesentlichen in Längsrichtung des Mantels 2' erstreckenden Stegelementen gebildet, die nach Art einer Haarnadel ausgebildet sind. Der Wendebereich bildet dabei jeweils das freie Ende des Stützelements 6', 7', 13.1' bzw. 13.2'.

Gestaltung und Anordnung der Stützelemente 6', 7', 13.1' bzw. 13.2' soll hier stellvertretend am Beispiel des Stützelements 6' erläutert werden. Die Schenkel 6.1' und 6.2' des Stützelements 6' sind, so am Steg 14' bzw. am Mantel 2' angeschlossen, daß sich die Anschlußpunkte 6.3' und 6.4' bei der Expansion der Stentstruktur voneinander entfernen. Hierdurch wird zum einen das haarnadelartige Stützelement 6' in der Mantelfläche von der dem Wendebereich 6.5' abgewandten Seite her aufgebogen, woraus eine Vergrößerung des durch sie abstützbaren Gefäßbereichs resultiert. Zum anderen entfernen sich hierdurch auch die freien Enden der Stützelemente 6' und 13.1' bzw. 7' und 13.2' in Umfangsrichtung voneinander, woraus eine gleichmäßigere Verteilung der Stützelemente über den abzustützenden Gefäßübergang resultiert.

Der dem Steg 14' zugewandte erste Schenkel 6.1' ist dabei kürzer als der dem benachbarten Stützelement 13.1' zugewandte zweite Schenkel 6.2', so daß die Anschlußpunkte 6.3' und 6.4' in Längsrichtung des Mantels 2' voneinander beabstandet sind. Im implantierten Zustand ist somit bei um die Verbindungslinie der Anschlußpunkte in den abzweigenden Gefäßastgeklappten Stützelementen 6', 7', 13.1' bzw. 13.2' ein der ellipsenartigen Kontur des Gefäßübergangs im wesentlichen angepaßter Durchgang in das abzweigende Gefäß freigegeben. Hierdurch ist eine gleichmäßige Abstützung des Gefäßübergangs sichergestellt.

Aufgrund der Neigung der Verbindungslinie der Anschlußpunkte zur Umfangsrichtung des Mantels verlaufen die in den abzweigenden Gefäßast geklappten Stützelemente 6', 7', 13.1' bzw. 13.2' geneigt zur Längsrichtung des abzweigenden Gefäßastes, wodurch sich die Abstützung der Gefäßwandung durch die Stützelemente 6', 7', 13.1' bzw. 13.2' im Bereich des Gefäßüberganges in vorteilhafter Weise über einen größeren Umfangsbereich des abzweigenden Gefäßastes erstreckt. Dabei ist die Neigung zur Längsachse des abzweigenden Gefäßastes damit der abgestützte Umfangsbereich umso größer, je stärker die Verbindungslinie der Anschlußpunkte zur Umfangsrichtung geneigt verläuft.

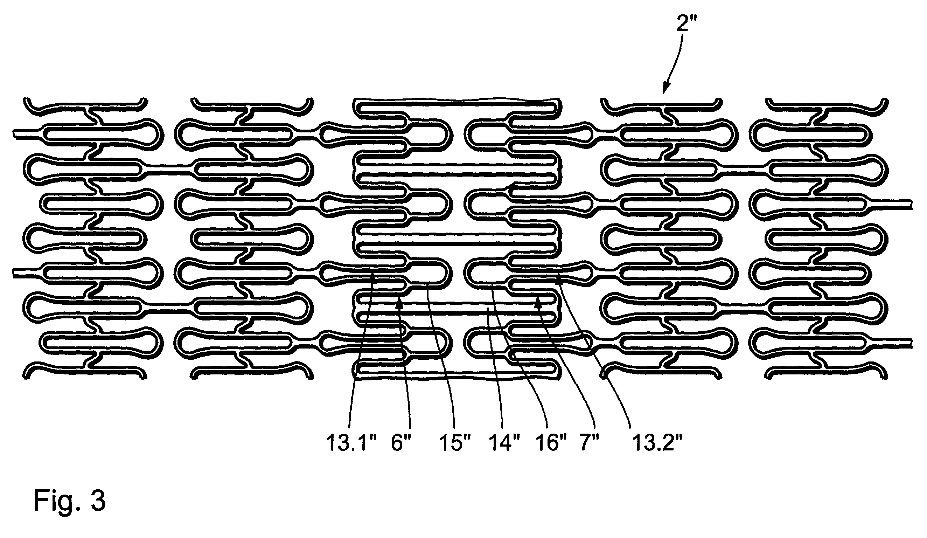

Figur 3 zeigt eine Abwicklung der Mantelfläche einer weiteren Ausführung eines erfindungsgemäßen Stents, die im wesentlichen der Ausführung aus Figur 2 gleicht, so daß hier lediglich auf die Unterschiede eingegangen werden soll.

Der Unterschied liegt dabei darin, daß die freien Enden der Stützelemente 6'' und 13.1'' über ein stegartiges bogenförmiges Verbindungselement 15'' und die freien Enden der Stützelemente 7'' und 13.2'' über ein stegartiges bogenförmiges Verbindungselement 16'' verbunden sind. Das bogenförmige Verbindungselement 15'' bzw. 16'' weist dabei eine Bogenlänge auf, die ausreicht, um eine Abstandsänderung zwischen den freien Enden der Stützelemente 6'' und 13.1'' bzw. 7'' und 13.2'' beim Expandieren des Stents und beim anschließenden Umklappen der Stützelemente in den abzweigenden Gefäßast auszugleichen. Im umgeklappten Zustand stützt das Verbindungselement 15'' bzw. 16'' dann in vorteilhafter Weise den Umfangsbereich der Gefäßwandung ab, der zwischen den freien Enden der Stützelemente 6'' und 13.1'' bzw. 7'' und 13.2'' liegt. Beim Implantieren der oben beschriebenen Stents wird unter Bezugnahme auf Figur 1 vorzugsweise in der im folgenden beschriebenen Weise verfahren.

Zunächst wird der Stent 1 im Bereich der Gefäßverzweigung 4 so positioniert, daß der Verzweigungsabschnitt 5 im Bereich der Gefäßverzweigung 4 angeordnet ist. Hierzu wird ein Ballonkatheter 9 mit einem Ballon 12 verwendet. Dieser Ballon 12 weist dabei zwei voneinander getrennte Kammern 12.1 und 12.2 auf, zwischen denen sich eine Austrittsöffnung für einen im Katheterschaft 9.1 geführten Führungsdraht 8 befindet.

Der Führungsdraht 8 wird zur Unterstützung des Positioniervorganges durch eine entsprechende Öffnung im Bereich des Verzweigungsabschnittes 5 des hierbei noch im wesentlichen unverformten Stents 1 seitlich aus dem Stent heraus und in den abzweigenden Gefäßast 3.1 geführt.

Durch Befüllen der Kammern 12.1 und 12.2 über die Befüllöffnungen 12.3 bzw. 12.4 im Katheterschaft 9.1 mit einem druckbeaufschlagten Fluid werden diese expandiert und damit der auf dem Ballon 12 sitzende Stent 1 unter plastischer Verformung aufgeweitet, so daß er das Gefäß 3 nach Entfernen des Ballons 12 dauerhaft aufgeweitet hält.

Nach diesem Expandieren des Mantels 2 des Stents 1 werden die Stützelemente 6 und 7 mittels des Führungsdrahtes 8 in Richtung der Pfeile 10 bzw. 11 radial aus der Mantelfläche 2.3 heraus unter plastischer Verformung in den abzweigenden Gefäßast 3.1 geklappt, um die Gefäßwandung im Bereich der Gefäßverzweigung 4 abzustützen.

Es versteht sich jedoch, daß der erfindungsgemäße Stent auch in anderer Weise, insbesondere unter Verwendung eines anders ausgebildeten Ballonkatheters, mit dem Ergebnis einer sehr guten Abstützung des Gefäßüberganges implantiert werden kann.

Die Erfindung beschränkt sich in ihrer Ausführung nicht auf die vorstehend angegebenen bevorzugten Ausführungsbeispiele. Vielmehr ist eine Anzahl von Varianten möglich, welche von der dargestellten Lösung auch bei grundsätzlich anders gearteten Ausführungen Gebrauch macht.

## Patentansprüche

1. Stent, insbesondere Koronarstent, zur Implantation im Bereich von Gefäßverzweigungen (4), mit einem rohrförmigen Mantel (2) mit einem proximalen und einem distalen Ende, wobei im implantierten Zustand das proximale Ende (2.1; 2.1') proximal der Gefäßverzweigung (4) und das distale Ende (2.2; 2.2') distal der Gefäßverzweigung (4) angeordnet ist, und wenigstens einem am Umfang des Mantels (2) angeordneten, zur Freigabe eines Durchgangs in den abzweigenden Gefäßast (3.1) vorgesehenen Verzweigungsabschnitt (5; 5'; 5''), der im implantierten Zustand im Bereich der Gefäßverzweigung angeordnet ist, wobei der Verzweigungsabschnitt (5; 5'; 5'') wenigstens ein zum Abstützen des Gefäßübergangs vorgesehenes, im wesentlichen radial aus der Mantelfläche herausklappbares erstes Stützelement (6; 6'; 6'') umfaßt, das sich aus einer ersten Richtung in den Verzweigungsabschnitt (5; 5'; 5'') hinein erstreckt,dadurch gekennzeichnet, daß der Verzweigungsabschnitt (5; 5'; 5'') wenigstens ein zum Abstützen des Gefäßübergangs vorgesehenes, im wesentlichen radial aus der Mantelfläche herausklappbares zweites Stützelement (7; 7'; 7'') umfaßt, das aus einer zweiten, anderen als der ersten Richtung in den Verzweigungsabschnitt (5; 5'; 5'') hinein erstreckt.

2. Stent nach Anspruch 1, dadurch gekennzeichnet, daß die zweite Richtung der ersten Richtung im wesentlichen entgegengesetzt ist.

3. Stent nach Anspruch 1 oder 2, gekennzeichnet durch wenigstens ein drittes, das zu Stützelement (6', 7'; 6'', 7'') benachbart angeordnet ist, Stützelement (13.1', 13.2'; 13.1'', 13.2'').

4. Stent nach Anspruch 2 und 3, dadurch gekennzeichnet, daß das dritte Stützelement (13.1', 13.2'; 13.1'', 13.2'') aus einer dritten, quer zur ersten und zweiten Richtung verlaufenden Richtung in den Verzweigungsabschnitt (5; 5'; 5'') hinein erstreckt.

5. Stent nach Anspruch 4, gekennzeichnet durch wenigstens ein zu dem ersten und zweiten Stützelement (6', 7'; 6'', 7'') quer zur ersten Richtung benachbart angeordnetes viertes Stützelement (13.1', 13.2'; 13.1'', 13.2'').

6. Stent nach einem der vorhergehenden Ansprüche, dadurch gekennzeichnet, daß das zweite Stützelement (7'; 7'') zu dem ersten Stützelement (6'; 6'') in der ersten Richtung im wesentlichen fluchtend angeordnet ist.

7. Stent nach einem der vorhergehenden Ansprüche, dadurch gekennzeichnet, daß die Stützelemente (6, 7; 6', 7', 13.1', 13.2'; 6'', 7'', 13.1'', 13.2'') langgestreckt ausgebildet sind.

8. Stent nach einem der vorhergehenden Ansprüche, dadurch gekennzeichnet, daß die Stützelemente (6', 7', 13.1', 13.2'; 6'', 7'', 13.1'', 13.2'') von sich im wesentlichen parallel zur ersten Richtung erstreckenden Stegelementen gebildet sind.

9. Stent nach Anspruch 8, dadurch gekennzeichnet, daß der Verlauf der Stegelemente im wesentlichen nach Art einer Haarnadel ausgebildet ist, wobei der Wendebereich (6.5') das freie Ende des Stützelements (6') bildet.

10. Stent nach einem der vorhergehenden Ansprüche, dadurch gekennzeichnet, daß quer zur ersten Richtung aneinander angrenzende Stützelemente (6'', 13.1''; 7'', 13.2'') an ihrem freien Ende über wenigstens ein, insbesondere bogenartig ausgebildetes, Verbindungselement (15''; 16'') verbunden sind.

11. Stent nach einem der vorhergehenden Ansprüche, dadurch gekennzeichnet, daß die Stützelemente (6', 7', 13.1', 13.2'; 6'', 7'', 13.1'', 13.2'') derart ausgebildet und angeordnet sind, daß der Verzweigungsabschnitt (5'; 5'') im implantierten Zustand bei zum Abstützen des Gefäßübergangs radial aus der Mantelfläche geklappten Stützelementen (6', 7', 13.1', 13.2'; 6'', 7'', 13.1'', 13.2'') einen der Kontur des Gefäßübergangs im wesentlichen angepaßten Durchgang in der Mantelfläche freigibt.

12. Stent nach einem der vorhergehenden Ansprüche, dadurch gekennzeichnet, daß er eine Anzahl Verzweigungsabschnitte (5'; 5'') aufweist.

13. Stent nach Anspruch 12, dadurch gekennzeichnet, daß wenigstens ein Teil der Verzweigungsabschnitte (5'; 5'') in einem Ringbereich des Mantels (2'; 2'') in Umfangsrichtung des Mantels (2'; 2'') verteilt angeordnet sind.

14. Stent nach Anspruch 13, dadurch gekennzeichnet, daß die Verzweigungsabschnitte (5'; 5'') in dem Ringbereich unmittelbar aneinander anschließend angeordnet sind.

15. Verfahren zum Implantieren eines Stents nach einem der vorhergehenden Ansprüche, dadurch gekennzeichnet, daß die Stützelemente (6, 7; 6', 7', 13.1', 13.2'; 6'', 7'', 13.1'', 13.2'') nach dem Expandieren des Mantels (2; 2'; 2'') durch ein an den Verzweigungsabschnitt (5; 5'; 5'') herangeführtes Betätigungsmittel (8), insbesondere einen Führungsdraht, radial aus der Mantelfläche heraus in die Gefäßverzweigung (4) hinein geklappt werden.

16. Verfahren nach Anspruch 15, dadurch gekennzeichnet, daß als Betätigungsmittel ein zum Positionieren des Stents vor der Expansion vorgesehener Führungsdraht (8) verwendet wird.

17. Verfahren nach Anspruch 16, dadurch gekennzeichnet, daß zum Expandieren des Stents ein Ballonkatheter verwendet wird, der an seinem distalen Ende einen proximalen und einen davon beabstandeten distalen Ballon (12.1, 12.2) aufweist, zwischen denen der das Betätigungsmittel bildende Führungsdraht (8) aus dem Katheterschaft austritt.
